# FASCICULE DE BREVET EUROPEEN

(11) **EP 1 666 582 B1**
(45) Date de publication et mention de la délivrance du brevet: **01.07.2009**
(21) Numéro de dépôt: 05292381.0
(22) Date de dépôt: 09.11.2005
(51) Int. Cl.: C12M 1/26

(54) **Dispositif de prélèvement de carottes pour tissue array**
Vorrichtung zur Entnahme von Kernproben für Gewebe-Array
Core sampling device for tissue array

(30) Priorité: 02.12.2004 FR 0412776
(43) Date de publication de la demande: 07.06.2006
(73) Titulaire: Alphelys, 78370 Plaisir (FR)
(72) Inventeur: Chaumat, Pierre, 78340 Les Clayes sous Bois (FR)
(74) Mandataire: Célanie, Christian

(56) Documents cités:
- WO-A-03/010280
- US-A- 6 103 518
- US-A1- 2002 146 813
- US-A1- 2003 017 446

## Description

Le secteur technique de la présente invention est celui des dispositifs permettant de produire des « tissue array ».

Le « tissue array » est une technique de montage de coupes des tissus biologiques sur lame de microscope en vue de leur analyse visuelle ou électronique. La technique du « tissue array » autorise le montage d'un grand nombre de coupes de tissus sur une même lame contrairement à la technique traditionnellement pratiquée dans les laboratoires de pathologie avec laquelle une à trois coupes peuvent être montées ensemble.

La technique du « tissue array » consiste à prélever une ou plusieurs carottes dans plusieurs dizaines, voire plusieurs centaines de blocs différents contenant des prélèvements tissulaires soit inclus dans de la paraffine, soit congelés. L'ensemble des carottes est ensuite assemblé dans un bloc de paraffine ou dans un milieu d'inclusion congelé dans lequel ont été pratiqués des évidements.

La technique du « tissue array » est bien connue et il n'est pas nécessaire de la décrire plus en détail.

On peut se reporter par exemple au brevet US-6103518 qui décrit un dispositif de carottage. La réalisation proposée est complexe et fondée sur un bras pivotant de manière à amener successivement dans une position de travail deux poinçons de prélèvement. La rotation du poinçon à l'autre contraint à un alignement parfait des deux poinçons au-dessus de la position de carottage. De plus, la position de la plate-forme porte blocs donneurs doit être modifiée à chaque opération, ce qui rend l'utilisation d'un tel dispositif peu commode et lent.

Le but de la présente invention est de fournir un dispositif de carottage de conception simple, de mise en oeuvre facile pour l'utilisateur et permettant de remédier à ces inconvénients.

L'invention a donc pour objet un dispositif de prélèvement de carottes destiné à la construction de «tissue array» du type comportant un poinçon de carottage pour la réalisation d'évidements dans un ou plusieurs blocs dits receveurs, un poinçon de prélèvement de carottes d'échantillon et un moyen d'éjection dans un ou plusieurs blocs receveurs en paraffine ou en tout milieu congelé ou non des carottes prélevées, caractérisé en ce que le poinçon de carottage est monté de manière sensiblement coaxiale dans le poinçon de prélèvement, le poinçon de prélèvement étant en position externe, les deux poinçons étant mobiles en translation et/ou rotation l'un par rapport à l'autre, et le moyen d'éjection étant agencé pour éjecter les carottes de chaque poinçon.

Selon une caractéristique de l'invention, le moyen d'éjection est disposé dans le poinçon de prélèvement.

Selon une autre caractéristique de l'invention, le moyen d'éjection se présente sous la forme d'une tige.

Selon encore une caractéristique de l'invention, le diamètre externe de la tige est proche du diamètre interne du poinçon de prélèvement.

Selon encore une caractéristique de l'invention, les poinçons et le moyen d'éjection sont mobiles en translation et en rotation indépendamment les uns par rapport aux autres.

Selon encore une caractéristique de l'invention, le diamètre interne du poinçon de carottage correspond sensiblement au diamètre externe de la carotte prélevée dans le bloc donneur.

Selon encore une caractéristique de l'invention, le poinçon de prélèvement présente un diamètre interne correspondant sensiblement au diamètre externe de la carotte prélevée dans le bloc receveur.

Selon encore une caractéristique de l'invention, les poinçons présentent un bord aiguisé, destiné à faciliter la pénétration desdits poinçons dans la paraffine ou le bloc congelé ainsi que l'extraction des carottes.

Selon encore une caractéristique de l'invention, le dispositif comprend un système optique et une première interface logiciel permettant le paramétrage de la ou des positions de carottage sur un bloc donneur, une seconde interface logiciel pour le paramétrage des positions des carottes sur le ou les blocs receveurs, et un système d'identification positive des blocs donneurs et receveurs.

Un tout premier avantage du dispositif selon l'invention réside dans le fait qu'elle permet aux pathologistes d'étudier un grand nombre de patients simultanément avec un budget restreint et dans un délai limité. Cela permet donc de largement accélérer les progrès de la connaissance dans la recherche vers de nouvelles méthodologies pronostiques, diagnostiques et donc de nouveaux traitements.

D'autres caractéristiques, détails et avantages de l'invention ressortiront plus clairement de la description donnée ci-après à titre indicatif en relation avec des dessins dans lesquels :
- la figure 1 représente une vue schématique du dispositif selon l'invention,

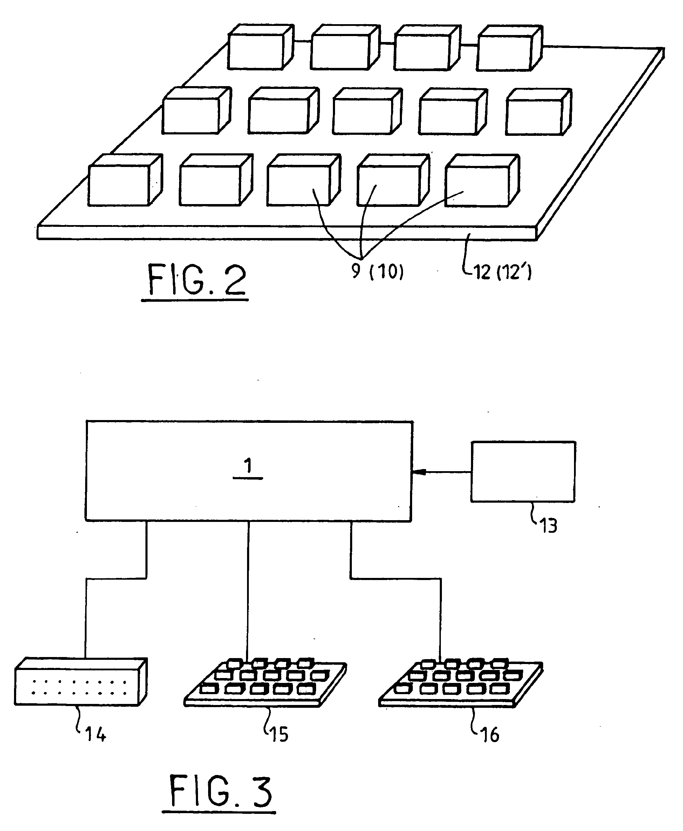

- la figure 2 montre un exemple de réalisation d'une plate-forme porte blocs, et
- la figure 3 illustre un exemple de réalisation d'un système de carottage.

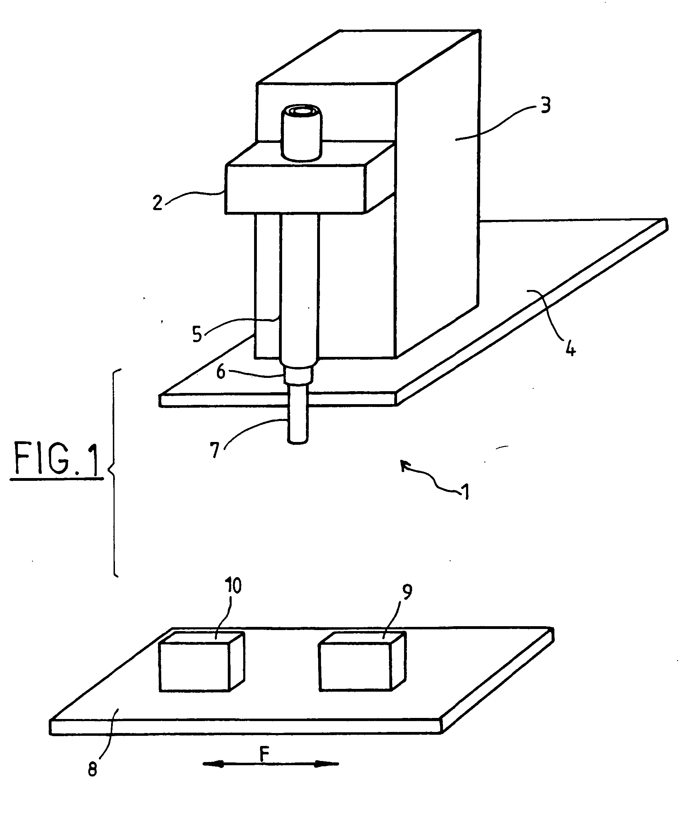

Sur la figure 1, on a représenté un dispositif de carottage 1 monté sur un support 2 lui-même solidaire d'un bâti 3 fixé sur une platine support 4 destinée à supporter l'ensemble des éléments nécessaires au fonctionnement du dispositif, telle une centrale hydraulique ou électrique nécessaire à la commande des différents éléments comme cela sera expliqué ci-après. Il comporte classiquement un poinçon de carottage 6 et un poinçon de prélèvement 5.

Suivant l'invention, ces deux poinçons 5 et 6 sont disposés alignés sur un même axe longitudinal en définissant un poinçon interne et un poinçon externe. Dans la suite de la description, on parlera du poinçon externe 5 et du poinçon interne 6. Le support 2 reçoit le poinçon externe 5, le poinçon interne 6 et un éjecteur 7. Ces trois pièces sont montées alignées sur un même axe et étant coulissant l'une part rapport à l'autre. Les poinçons 5 et 6 se présentent sous la forme de deux tubes, le poinçon interne 6 étant inséré dans le poinçon externe 5. Ces deux poinçons coulissent suivant un mouvement de va et vient indépendamment l'un de l'autre. L'éjecteur 7 se présente sous la forme d'une pièce cylindrique pleine insérée dans le poinçon interne. L'ensemble de ces trois pièces constitue ainsi un ensemble compact permettant d'assurer toutes les fonctionnalités du dispositif 1. Par ailleurs ces trois pièces sont rendues mobiles soit manuellement à l'aide de systèmes à crémaillères soit à l'aide de la centrale hydraulique évoquée précédemment.

Sur la figure, on a également représenté une plaque support 8 sur laquelle sont fixés deux blocs de carottage 9 et 10. Le bloc 9 est constitué par exemple d'un échantillon de tissus dans lequel on va procéder à des prélèvements. Ce bloc 9 sera qualifié de bloc donneur. Le bloc 10 est un bloc vierge destiné à recevoir les échantillons prélevés dans différents blocs donneurs. Le bloc 10 sera qualifié de bloc receveur. Ces deux blocs sont classiquement des blocs de paraffine ou des blocs congelés.

Avantageusement, le support 2 peut être mobile et les plaques de blocs sont fixes ou l'inverse.

Le dispositif de carottage 1 selon l'invention est utilisé de la manière suivante. Bien entendu, le dispositif 1 est préférentiellement utilisé en position verticale telle qu'elle apparaît dans le plan de la figure.

On commande tout d'abord l'escamotage de l'éjecteur 7 et du poinçon externe 5 afin de libérer au moins partiellement le poinçon interne 6. On amène le bloc receveur 10 en fixant précisément sa position à l'aide d'un système de repérage classique. On prélève tout d'abord une carotte dans le bloc receveur 10 à l'aide du poinçon interne 6 pour réserver un évidement d'insertion de l'échantillon de tissu. Pendant cette phase, le poinçon externe 5 et l'éjecteur 7 sont escamotés complètement comme indiqué ci-dessus.

On commande une rotation du poinçon interne 6 pour expulser la carotte prélevée du bloc receveur. Cette rotation casse la base de la carotte qui peut donc être extraite sans difficulté. La carotte est éliminée en faisant coulisser l'éjecteur 7 dans le poinçon interne 6.

On prélève ensuite une carotte dans un bloc donneur 9 à l'aide du poinçon externe 5 en amenant ce bloc à la verticale des poinçons. Pour ce faire, le poinçon interne 6 et l'éjecteur 7 sont escamotés complètement vers le support 2 manuellement à l'aide d'une crémaillère ou automatiquement à l'aide de la centrale hydraulique ou électrique. On sépare la carotte d'échantillon du bloc donneur 9 par la rotation du poinçon externe 5.

On transfère alors la carotte prélevée du bloc donneur 9 dans le bloc receveur 10 après avoir ramené celui-ci dans la position repérée initialement en faisant correspondre l'évidement obtenu précédemment avec la position du poinçon externe 5. La carotte prélevée dans le bloc donneur 9 est alors transférée dans cet évidement par le coulissement de l'éjecteur 7 et du poinçon interne 6 dans le poinçon externe 5.

En contrôlant la hauteur à laquelle l'éjecteur 7 s'arrête, on détermine la profondeur à laquelle la carotte est introduite dans l'évidement.

On recommence cette séquence d'opérations autant de fois que nécessaire pour obtenir le nombre de carottes désirées dans le bloc receveur 10 en utilisant différents blocs donneurs 9.

Avantageusement, le dispositif de carottage 1 selon l'invention est.inclus dans un ensemble non représenté ici, qui permet de motoriser les mouvements de translation et de rotation des poinçons 5 et 6 et de l'éjecteur 7 ainsi que le positionnement des poinçons au-dessus des blocs donneurs et receveurs.

Cet appareil, dénommé selon le vocable anglais « tissue arrayér », permet de construire des « tissue arrays » à partir de blocs donneurs inclus dans de la paraffine ou à partir de blocs congelés. Dans ce dernier cas, les poinçons sont eux-mêmes réfrigérés pour préserver l'état congelé des carottes lors du prélèvement et du transfert.

Sa structure peut se présenter de la manière suivante.

Selon une réalisation, l'appareil 1 comporte un plateau porteur 12 destiné à supporter des blocs donneurs 9 et un second plateau porteur 12' destiné à porter les blocs receveurs 10. Ces plateaux 12 et 12' peuvent être retirés de l'appareil 1 pour être chargés en blocs donneurs et receveurs. L'appareil 1 peut comporter également une plate-forme comportant des moyens de positionnement pour les plateaux porteurs. Cette plate-forme peut intégrer une ou plusieurs positions pour des plateaux à blocs donneurs en fonction de la capacité souhaitée par l'utilisateur. Chaque position comporte un système de réfrigération pour maintenir congelés les blocs donneurs et receveurs lors de la construction de « tissue arrays » congelés si on opère à partir de blocs congelés.

L'appareil comprend un dispositif de carottage tel que décrit précédemment placé sur un ensemble motorisé permettant le déplacement au micron près selon les trois axes dans l'espace afin de placer le système de carottage en des positions précises et paramétrées au-dessus des blocs donneurs et receveurs. La motorisation permet également la rotation indépendante de chaque poinçon, ainsi que le coulissement de chaque poinçon et de l'éjecteur.

L'appareil peut également comprendre un système optique 13 et une première interface logicielle 14 permettant le paramétrage de la ou des positions de carottage sur le bloc donneur, et une seconde interface logicielle 15 pour le paramétrage des positions des carottes sur le ou les blocs receveurs.

L'appareil peut intégrer un système d'identification positive 16 des blocs donneurs et receveurs avant chaque carottage (lecture de code-barres, magnétiques, etc...) pour éviter toute erreur.

Il peut encore comprendre aussi un système de détection du prélèvement et de la longueur des carottes et de gestion associée des erreurs.

Enfin, il peut être enclos dans une enceinte close afin d'empêcher les utilisateurs d'intervenir durant les mouvements du système de carottage et de protéger les échantillons durant les opérations et à les maintenir en atmosphère sèche pour éviter la formation de condensation et de givre sur les blocs, les structures et les poinçons en cas de construction de « tissue array » à partir de blocs donneurs congelés.

## Revendications

1. Dispositif de prélèvement de carottes (1) destiné à la construction de « tissue array » du type comportant un poinçon de prélèvement (5) de carottes d'échantillon, un poinçon de carottage (6) pour la réalisation d'évidements dans un ou plusieurs blocs dits receveur et un moyen d'éjection (7) des carottes prélevées dans un ou plusieurs blocs receveurs (10) en paraffine ou en tout milieu congelé ou non, **caractérisé en ce que** le poinçon de carottage (6) est monté de manière sensiblement coaxiale dans le poinçon de prélèvement (5), le poinçon de prélèvement étant en position externe, les deux poinçons (5, 6) étant mobiles en translation et/ou rotation l'un par rapport à l'autre, et le moyen d'éjection étant agencé pour éjecter les carottes de chaque poinçon.

2. Dispositif selon la revendication 1, **caractérisé en ce que** le moyen d'éjection (7) est disposé dans le poinçon interne (6).

3. Dispositif selon la revendication 2, **caractérisé en ce que** le moyen d'éjection (7) se présente sous la forme d'une tige.

4. Dispositif selon la revendication 3, **caractérisé en ce que** le diamètre externe de la tige est proche du diamètre interne du poinçon interne (6).

5. Dispositif selon l'une quelconque des revendications précédentes, **caractérisé en ce que** les poinçons (5, 6) et le moyen d'éjection (7) sont mobiles en translation et en rotation indépendamment les uns par rapport aux autres.

6. Dispositif selon l'une quelconque des revendications précédentes, **caractérisé en ce que** le diamètre interne du poinçon externe (5) correspond sensiblement au diamètre externe de la carotte prélevée dans le bloc donneur (9).

7. Dispositif selon l'une quelconque des revendications précédentes, **caractérisé en ce que** le poinçon interne (6) présente un diamètre interne correspondant sensiblement au diamètre externe de la carotte prélevée dans le bloc receveur (10).

8. Dispositif selon l'une des revendications 2 à 7, **caractérisé en ce que** les poinçons (5, 6) présentent un bord aiguisé, destiné à faciliter la pénétration desdits poinçons dans la paraffine ou le bloc congelé ainsi que l'extraction des carottes.

9. Dispositif selon l'une quelconque des revendications précédentes, **caractérisé en ce qu'**il comprend un système optique (13) et une première interface logiciel (14) permettant le paramétrage de la ou des positions de carottage sur un bloc donneur (9), une seconde interface logiciel (15) pour le paramétrage des positions des carottes sur le ou les blocs receveurs (10), et un système d'identification positive (16) des blocs donneurs (9) et receveurs (10).

## Claims

1. A core sampling device (1) intended to assemble tissue arrays of the type incorporating a core sampling punch (5), a core excision punch (6) intended to make recesses in one or several so-called receiver blocks, and means (7) to expel the sample cores into one or several paraffin receiver blocks (9, 10) or into any frozen milieu or not, **characterized by** the fact that the core excision punch (6) is mounted substantially coaxially in the core sampling punch (5), the sampling punch being in the external position, both punches (5, 6) being able to move in translation and/or rotation with respect to one another, and the ejection means being arranged to as to expel the cores from each punch.

2. Device according to Claim 1, **characterized by** the fact that the ejection means (7) are positioned in the internal punch (6).

3. Device according to Claim 2, **characterized by** the fact that the ejection means (7) are in the form of a rod.

4. Device according to Claim 3, **characterized by** the fact that the external diameter of the rod is close to the internal diameter of the internal punch (6).

5. Device according to any one of the above Claims, **characterized by** the fact that the punches (5, 6) and ejection means (7) are able to move in translation and in rotation independently of one another.

6. Device according to any one of the above Claims, **characterized by** the fact that the internal diameter of the external punch (5) substantially corresponds to the external diameter of the core extracted from the donor block (9).

7. Device according to any one of the above Claims, **characterized by** the fact that the internal punch (6) has an internal diameter that substantially corresponds to the external diameter of the core extracted from the receiver block (10).

8. Device according to one of Claims 2 to 7, **characterized by** the fact that the punches (5, 6) have a sharp edge intended to facilitate their penetration into the paraffin or the frozen block as well as to facilitate the extraction of the cores.

9. Device according to any one of the above Claims, **characterized by** the fact that it comprises an optical system (13) and a first software interface (14) enabling the core extraction positions to be parametered on a donor block (9), a second software interface (15) to parameter the core positions on the receiver block or blocks (9), and a positive identification system (16) for the donor (9) and receiver (10) blocks.

## Patentansprüche

1. Vorrichtung zur Aufnahme von Bohrkernen (1), die zur Herstellung von "Tissue Array" vorgesehen ist, umfassend einen Dorn zur Aufnahme (5) von Probebohrkernen, einen Dorn zur Kernbohrung (6) zur Ausführung von Aussparungen in einem oder mehreren so genannten Aufnehmer-Blöcken und ein Mittel zum Ausstoßen (7) der aufgenommenen Bohrkerne in einen oder mehrere Aufnehmer-Blöcke (10) aus Paraffin oder aus irgendeinem erstarrten oder nicht erstarrten Medium, **dadurch gekennzeichnet, dass** der Dorn zur Kernbohrung (6) in im wesentlichen koaxialer Weise im Aufnahmedorn (5) montiert ist, wobei der Aufnahmedorn sich in äußerer Position befindet, die beiden Dorne (5, 6) verschiebbar und/oder drehbar zueinander beweglich sind und das Ausstoßmittel dafür eingerichtet ist, um die Bohrkerne von jedem Dorn auszustoßen.

2. Vorrichtung nach Anspruch 1, **dadurch gekennzeichnet, dass** das Ausstoßmittel (7) im inneren Dorn (6) angeordnet ist.

3. Vorrichtung nach Anspruch 2, **dadurch gekennzeichnet, dass** das Ausstoßmittel (7) sich in der Form einer Stange darstellt.

4. Vorrichtung nach Anspruch 3, **dadurch gekennzeichnet, dass** der äußere Durchmesser der Stange nah an dem inneren Durchmesser des inneren Dorns (6) liegt.

5. Vorrichtung nach irgendeinem der vorhergehenden Ansprüche, **dadurch gekennzeichnet, dass** die Dorne (5, 6) und das Ausstoßmittel (7) unabhängig zueinander verschiebbar und drehbar beweglich sind.

6. Vorrichtung nach irgendeinem der vorhergehenden Ansprüche, **dadurch gekennzeichnet, dass** der innere Durchmesser des äußeren Dorns (5) im wesentlichen dem äußeren Durchmesser des aufgenommenen Bohrkerns in dem Geber-Block (9) entspricht.

7. Vorrichtung nach irgendeinem der vorhergehenden Ansprüche, **dadurch gekennzeichnet, dass** der innere Dorn (6) einen inneren Durchmesser aufweist, der im wesentlichen dem äußeren Durchmesser des aufgenommenen Bohrkerns in dem Aufnehmer-Block (10) entspricht.

8. Vorrichtung nach irgendeinem der Ansprüche 2 bis 7, **dadurch gekennzeichnet, dass** die Dorne (5, 6) einen geschärften Rand aufweisen, der dafür vorgesehen ist, das Eindringen der genannten Dorne in das Paraffin oder den erstarrten Block sowie die Entfernung der Bohrkerne zu erleichtern.

9. Vorrichtung nach irgendeinem der vorhergehenden Ansprüche, **dadurch gekennzeichnet, dass** sie ein optisches System (13) und eine erste Software-Schnittstelle (14), welche die Parametrierung der Position oder Positionen der Kernbohrung an dem Geber-Block (9) ermöglicht, eine zweite Software-Schnittstelle (15) für die Parametrierung der Positionen der Bohrkerne an dem oder den Aufnehmer-Blöcken (10) und ein System zur positiven Identifizierung (16) der Geber (9)- und Aufnehmer-Blöcke (10) umfasst.
